# EUROPEAN PATENT APPLICATION

(11) **EP 4 115 855 A1**
(43) Date of publication of application: **11.01.2023**
(21) Application number: 21315125.1
(22) Date of filing: 09.07.2021
(51) Int. Cl.: A61F 5/00

(54) **PYLORIC ANCHOR AND GASTRO-INTESTINAL TUBE**

(71) Applicant: BariaTek Medical, 75003 Paris (FR)
(72) Inventor: The designation of the inventor has not yet been filed
(74) Representative: Hepp Wenger Ryffel AG

(57) **Abstract**

A bypass tube (14) has a distal end for placement into the small intestine of a patient and a proximal end for placement near or at the pylorus. The tube comprising a pyloric anchor (16) including a gastric anchor (22) for anchoring the proximal end of the tube with respect to the pylorus to prevent distal migration. The gastric anchor comprises a tubular balloon (24) and a self-expanding structure (22a, 22b) for biasing a radially inner wall and/or a radially outer wall of the tubular balloon outwardly towards its enlarged form. The self-expanding structure comprises a first zone comprising an annular hub or collar (22a) with a first resistance to inward collapsing, and a second zone comprising a plurality of independently deflectable beams or limbs (22b) extending from the first zone, and having a smaller second resistance to inward collapsing. Also described are a pyloric anchor that twists around its axis when expanding, and a non-helical reinforcement of the flexible tube in the small intestine.

## Description

### Field of the Invention '

The present invention relates to the field of devices for insertion into the gastro-intestinal tract and for anchoring at the pylorus. In some non-limiting aspects, the device may be a bypass tube for bypassing a portion of the bowel, or a plug for plugging the pylorus.

### Background to the Invention

Various surgical techniques, and implants, have been proposed for treating obesity and diabetes. Surgical techniques include creation of gastric pockets and gastric bypasses of the stomach, duodenum and part of the jejunum. Bypass tubes or liners have been proposed for insertion into the gastro-intestinal tract, to bypass the duodenum and optionally part of the jejunum.

Technical challenges remain for many of these techniques. For example, the endoscopic placement and anchoring of bypass tubes remains challenging. One technique proposed is to anchor the tube in the vicinity of the pylorus. However, the stomach and intestine are subject to significant motion in normal bodily function. Muscular contractions of the stomach, including at the pyloric antrum, complicate maintaining the tube in position. The muscular contractions can be extreme in the case of, for example, the patient vomiting. Dislodgement either towards the duodenum, or into the stomach, can necessitate medical intervention to correct the position or to retrieve the bypass tube. Many existing proposals are compromised by the apparently conflicting need for secure anchoring, yet with atraumatic engagement with the body tissue to avoid the device causing tissue irritation.

It would be desirable to address and/or mitigate one or more of the above issues.

### Summary of the Invention

Aspects of the invention may be defined in the claims.

A first aspect of the disclosure provides a gastric anchor for anchoring a tube with respect to a pylorus of a patient, the tube having a distal end for placement within the small intestine and a proximal end for placement near or at the pylorus and/or within the stomach, the gastric anchor serving to prevent distal migration of the proximal end, the gastric anchor comprising a tubular balloon and a self-expanding structure, the gastric anchor having: a first end for seating on the gastric side of the pylorus; a first region having a first resistance to inward collapsing; and a second region having a second smaller resistance to inward collapsing, the first region being closer than the second region to the first end.

Such a configuration can provide a gastric anchor that has tailored resistances to collapsing. The second region can be relative conformable to accommodate contractions of the stomach and/or antrum and reduce risk of the contractions dislodging the anchor in a proximal direction. The second region can permit inward collapsing to a degree allowing digested food (e.g. chyme) to pass from the stomach to the duodenum. The first region can be relatively firm to ensure that gastric anchor will retain a sufficient size to obstruct distal migration through the pylorus even when the first region of the gastric anchor collapses at least partly inwardly under the influence of a stomach contraction.

In some embodiments, the different first and second resistances to collapsing are provided at least partly by the self-expanding structure. The self-expanding structure may comprise a first zone having first zone resistance to inward collapsing, and a second zone with a smaller second zone resistance to inward collapsing.

The self-expandable structure may have any desired form. For example, it may comprise a stent or stent-like structure. It may define a framework of struts and/or'cells.

In some embodiments, the first zone of the self-expanding structure may comprise an annular hub or collar, and/or the second zone of the self-expanding structure may comprise a plurality of independently deflectable limbs or beams extending from the first zone.

A closely related second aspect of the disclosure provides a gastric anchor for anchoring a tube with respect to a pylorus of a patient, the tube having a distal end for placement within the small intestine and a proximal end for placement near or at the pylorus and/or in the stomach, the gastric anchor serving to prevent distal migration of the proximal end, the gastric anchor comprising a tubular balloon and a self-expanding structure for biasing a radially innerwall and/or a radially outer wall of the tubular balloon outwardly, towards its enlarged form, the self-expanding structure comprising a first zone comprising an annular hub or collar, and a second zone comprising a plurality of independently deflectable beams or limbs extending from the first zone.

In either aspect, the self-expanding structure may be attached to and/or embedded within one of the walls, for example, the radially outer wall, to draw the wall into contact with the surrounding stomach wall.

In either aspect, the independently deflectable beams/limbs, where provided, can provide conformability, and/or the annular hub/collar, where provided, can provide greater structural resistance to inward bending or collapsing. Each independently deflectable beam/limb, where provided, may comprise single elon-' gate rib or strut, or it may comprise plural struts collectively defining the beam or limb shape.

Additionally or alternatively, in either aspect, the tubular balloon may have any desired exterior shape or profile for fitting within the pyloric antrum and/or stomach. Suitable shapes include, for example, a cup-shape, a tulip-shape, a bell-shape, a pear-shape, a tear-drop shape, a funnel-shape, an umbrellashape. The tubular balloon can includes an internal channel, and an entrance mouth for admitting chyme into the channel to pass through the anchor towards the pylorus. The balloon may serve to occupy space for gastric restriction. Additionally or alternatively, the balloon may take some of the load from the self-expanding structure, and reduce risk of the structure becoming fatigued, or fracturing, or causing a central gastro-duodenal obstruction.

The deflectable beams/limbs, where provided, can deflect inwardly to allow local inward collapsing of the balloon to accommodate stomach/antrum contractions, and permit chyme to pass to the duodenum, as explained above. The deflectable limbs/beams are biased outwardly to urge the balloon to re-expand, and to follow movement of the stomach wall, when the contraction is over.

The hub/collar, where provided, may comprise a plurality of struts defining a structure of cells. By way of example, the hub or collar may comprises at least one row of cells, optionally two rows, or more. The hub/collar may provide a stronger (e.g. radially stronger) structure than the deflectable beams/limbs, to ensure that the end of the gastric anchor closer to the pylorus remains sufficiently large to avoid accidentally migration through the pylorus and into the duodenum, even in the presence of strong stomach contractions. The axial length of the hub/collar can be dimensioned such that the beams/limbs may be positioned where highest antral motility strains occur.

Additionally or alternatively to any of the above, the self-expanding structure may have any desired form. For example, it may comprise a stent or stent-like structure. It may define a framework of struts and/or cells. Each independently deflectable beam/limb, where provided, may comprise single elongate rib or strut, or it may comprise plural struts collectively defining the beam or limb shape.

Additionally or alternatively to any of the above, the radial thickness of the tubular balloon may be generally constant around the axis of the balloon and/or the channel, but this is not essential.

Additionally or alternatively to any of the above, the balloon walls may be of substantially inelastic material, such that the balloon wall material does not stretch under normal inflation of the balloon.

Additionally or alternatively to any of the above, the balloon may have a volume in the range of 200mL - 500mL. The balloon may optionally serve as a gastric occupying component to reduce the volume of the stomach for food.

Additionally or alternatively to any of the above, at least the gastric anchor and optionally the pyloric anchor as a whole, is collapsible to a non-deployed configuration for introduction through the stomach towards the pylorus, and is expandable to a deployed configuration for anchor at or near the pylorus. The self-expandable structure, where provided,

Additionally or alternatively to any of the above, the balloon may be inflated with any suitable fluid, such as a gas (e.g. air) or a liquid (e.g. saline). In some embodiments, the inflation pressure is optionally not greater than 2 atm, optionally not greater than 1.75 atm, optionally not greater than 1.5 atm, optionally not greater than 1.25 atm, optionally about 1 atm (for example, which corresponds generally to pressure equilibrium with ambient pressure within the stomach).

A closely related third aspect of the disclosure provides a method of deploying a gastric anchor for anchoring a tube with respect to a pylorus of a patient, the tube having a distal end for placement within the small intestine and a proximal end for placement near or at the pylorus, the gastric anchor serving to prevent distal migration of the proximal end, the gastric anchor comprising a tubular balloon and a self-expanding structure, the method comprising the steps of:
(i) introducing the gastric anchor into the stomach near the pylorus;
(ii) inflating the tubular balloon with an inflation fluid to a pressure selected as one or more of: a pressure that does not prevent at least partial inward collapsing of the tubular balloon in response to stomach contractions; a pressure not greater than 2 atm; a pressure not greater than 1.75 atm; a pressure not greater than 1.5 atm; a pressure not greater than 1.25 atm; a pressure about 1 atm; and
(iii) sealing the inflation balloon after inflation to prevent escape of the inflation fluid.

A fourth aspect of the invention, optionally including any of the aforementioned aspects and features, provides an anchor (e.g. a pyloric anchor) for anchoring a device with respect to a pylorus of a patient. The anchor comprises a frame extending around an axis, the frame being transformable from a non-deployed configuration for introduction to the pylorus, towards a deployed configuration for anchoring at the pylorus. The frame is configured such that transformation from the non-deployed configuration towards the deployed configuration causes a first portion of the frame to at least partly twist around the axis relative to a second portion of the frame.

The first portion of the frame that twists may be an intermediate frame portion disposed, at least in the deployed configuration, between the second frame portion and a third portion of the frame.

A closely related fifth aspect of the disclosure, optionally in combination with any of the aspects and features described above, provides an anchor comprising a frame extending around an axis, the frame including a first intermediate portion disposed axially between second and third portions, the frame having a non-deployed configuration for introduction to the pylorus and a deployed configuration for anchoring at the pylorus and in which struts of the first portion are biased to extend predominantly in the same direction as one another twisting at least partly around the axis to torsionally couple the second portion to the third portion.

In the non-deployed configuration, struts of the first portion may be less twisted around the axis than in the deployed configuration, optionally extending substantially axially without twisting around the axis.

The frame may be deformable to the non-deployed configuration, and be self-biasing to transform from the non-expanded configuration to the expanded configuration.

Optionally, the second and third frame portions are anchor portions for engaging tissue on either side of the pylorus, and the first portion is a bridging portion for passing within the pylorus.

The first portion may cause relative angular displacement around the axis, between the second and third portions when the frame transforms towards the deployed configuration. Additionally or alternatively, transformation from the non-deployed configuration towards the deployed configuration causes the first and second portions of the frame to move axially closer to each other, optionally to bias at least one, optionally both, of the second and third portions to bear axially against the pylorus.

Such configurations can preload the frame, and enable loads and/or displacements to be converted between axial and rotational senses. The twisting action and/or torsional configuration can bias the second and third portions axially towards each other, to compress the pylorus axially. Axial displacement between the second and third portions can be accommodated by partial twisting (or partial untwisting) motion, without detracting from the anchoring effect.

Certain characteristics of the frame can also be used to tailor migration resistance. For example, one of the second and third portions may be more rigid than the other,,such that the morerigid portion may be expected to rotate locally less than the other. Optionally, the second portion in the duodenum may be more rigid than the third gastric portion in the stomach. Alternatively, the third gastric portion in the stomach may be more rigid than the second portion in the duodenum. Such configurations might lead to different efficiencies in migration resistance, allowing tailoring as desired.

In some embodiments, in the deployed configuration, the first portion defines a waist between the second and third portions. Twisting can increase a peripheral (e.g. circumferential) separation between two points on the frame, causing the frame to waist inwardly to a smaller lateral (e.g. radial) dimension to accommodate the increased peripheral (e.g. circumferential) separation.

In the deployed configuration of the frame, one of the first and second portions may optionally have a larger lateral dimension (e.g. diameter) and/or axial dimension (e.g. axial length) than the other of the portions.

The self-deploying frame may, for example, be made of a shape memory alloy, for example, an alloy comprising nickel (Ni) and titanium (Ti), optionally also comprising cobalt (Co). For example, the shape memory alloy may be or comprise nitinol. The frame may comprise a lattice of interconnected frame struts and/or a braided wire structure

The frame may be biased towards its deployed configuration (for example, by heat setting the frame in the deployed configuration), and be deformable to its non-deployed configuration for loading into, for example, a sheath. Upon removal from the sheath, the frame can self-deploy to the deployed configuration by virtue of a shape memory effect.

Configuration of frame using any of the above features can provide a versatile frame geometry that can be configured for different applications. The degree of inward waisting of the twisting first portion can be controlled by the degree of twist around the frame axis. The greater the angle of twist, the narrower the waist. The degree of twisting can be one of the parameters used during heat setting to determine the shape of the frame in the deployed configuration. For example, for a pyloric plug, the frame can be configured with a large twisting angle around the axis to substantially pinch the centre of the frame closed. Alternatively, for an anchor for a duodenal tube, for example, the frame can be configured with sufficient twist to form a waist between the first and second anchor regions, leaving open a sufficiently large through-passage to allow passage of chyme.

Optionally, the frame may be provided on its interior surface and/or its exterior surface, or be coated, with liner material. Suitable liner material may comprise biological tissue (for example, pericardial tissue) or synthetic polymeric material (for example, polyurethane or PTFE). The material may be provided in sheet form, film form, a coating, or as a woven fabric or a nonwoven fabric.

Liner material on the interior surface can provide an inner conduit, or an inner seal in the case of a pyloric plug. Liner material on the exterior surface can enhance atraumatic fitting of the anchor with respect to anatomical tissue at and/or on either side of the pylorus, and avoid direct contact between the frame and the anatomical tissue should this be desirable.

In addition to any of the foregoing, the disclosure also provides a device for fitting at least partly at a pylorus of a patient. The device can comprise an anchor including any one or more of the features above. The device may, optionally, be a pyloric plug, or a duodenal tube.

When the fourth and/or fifth aspect is used in combination with any of the first to third aspects above, the gastric anchor may correspond to, and/or constitute, the second and/or third portion of the fourth and/or fifth, aspect.

A closely related sixth aspect of the invention provides a method of deploying a pyloric anchor at a pylorus of a patient, the anchor optionally according to the fourth and/or fifth aspect, the anchor comprising a frame extending around an axis, the method comprising:
(i) introducing the pyloric anchor into the stomach and to the pylorus of a patient with the anchor in a non-deployed configuration; and
(ii) transforming the anchor from the non-deployed configuration to a deployed configuration, the step of transforming comprising causing a first portion of the frame to twist at least partly around an axis with respect to a second portion of the frame.

The step of causing twisting may comprise releasing a self-deploying frame to twist as the frame self-deploys from the non-deployed configuration to the deployed configuration. Alternatively, the step of causing the frame to twist may comprise forcibly twisting the frame by application of a twisting force to the frame.

A seventh aspect of the present disclosure, optionally in combination with any of the preceding aspects, comprises a bypass tube for placement into the small intestine of a patient, the bypass tube made of flexible material and having a longitudinal axis, the bypass tube having a radially compressed configuration for introduction, and a radially expanded configuration for functional implantation, the tube carrying a reinforcement element for reinforcing the flexible tube, the reinforcing element having a non-helical shape.

Use of a non-helical reinforcement element can avoid complications caused by a helical shape tending to lengthen axially when compressed radially, and foreshorten axially when expanding radially. Such changes in axial length can apply additional stresses to the flexible tube, especially when made of inelastic material.

In some embodiments, the reinforcement element has a shape comprising circumferentially expandable and collapsible rows, interconnected by at least one substantially axial connecting segment.

Each row may, for example, comprise a reinforcement pattern extending generally in a circumferential direction of the tube. The row may extend around the entire circumference (e.g. around 360° of the circumference), or only partially around the circumference (e.g. around about 180° of the circumference). Using a partial circumference may leave a portion of the flexible tube unreinforced, and able to conform to movements of the small intestine, for example, peristaltic movements for advancing chyme.

Each row may optionally have a zig-zag or sinuous reinforcement pattern. Such a pattern can be compressible and expandable in the circumferential direction, without changing axial dimension substantially.

The axial segment(s) also need not change length during radial compression and expansion, because the axial segment(s) is generally perpendicular to the direction of changing dimension.

The tube may have a length for extending from the pylorus into the duodenum, and optionally into the jejunum.

The reinforcement may serve to limit the free movement of the tube so as to avoid twisting or kinking of the tube. The reinforcement may be present throughout the tube and/or be restricted to individual regions; and the reinforcement may be in and/or on the tube. Twists in a bypass tube can limit the flow of gastric content toward the bowel; this in turn can lead to the discomfort of the patient. In cases of extreme twisting, the tube can become completely blocked and obstruct the passage of any gastric content. The reinforcement confers a certain firmness to the tube thus avoiding twisting and/or blockages due to kinking.

In some embodiments, the reinforcement may be made of any suitable material such as metal and preferably Nitinol. Said reinforcement may be situated in and/or on the tube.

In some embodiments the above reinforcement may comprise at least one repetitive pattern. Said repetitive pattern may be at least one continuous triangular pattern and/or sinusoidal pattern and said repetitive pattern may run along the circumference of the tube. The use the repetitive pattern allows the tube to compress circumferentially when necessary whilst ensuring firmness around the reinforcement.

The reinforcement may consist of at least one circumferential and/or partially circumferential row of repetitive reinforcement, for example, the reinforcement may expand over 380° or over 180° of the tube.

In a preferred embodiment the reinforcement structure may comprise at least one repetitive reinforcement pattern arranged in at least one row. Optionally, there may be a succession of rows the length of the tube, said rows may be spaced out so as to confer flexibility to the tube between the rows thus allowing the tube to adapt to muscular contractions all the while ensuring a sufficient support with the reinforced sections so as to avoid twisting or kinking of the tube.

In some embodiments sections of the repetitive reinforcement pattern running over at least at portion of the circumference of the tube may be positioned on alternate opposite sides.

In any of the forgoing aspects, use or optional use of a self-deploying and/or self-expanding structure and/or frame and/or reinforcement can avoid the need to forcibly deploy the structure or frame using a special delivery tool. It can also permit the devices and/or anchors to be easily retrievable after implantation when their function is no longer desired or when the device reaches the end of its functional life. It can also permit the devices and/or anchors and/or reinforcement to be deformable have resilience to better perform their function in the mobile environment of the stomach and small intestine. However, if preferred, a non-self-deploying material may be used for the frame or structure or reinforcement.

A self-deploying frame or structure may, for example, be made of a shape memory alloy, for example, an alloy comprising nickel (Ni) and titanium (Ti), optionally also comprising cobalt (Co). For example, the shape memory alloy may be or comprise nitinol. The frame may comprise a lattice of interconnected frame struts and/or a braided wire structure

By way of example, the duodenal side of a pyloric anchor may have an outer diameter in a range of about 3cm to about 4cm.

The gastric side of a pyloric anchor may have an outer diameter in a range of about 4cm to about 6cm, optionally about 5cm. The tubular balloon may have a radial wall thickness of about 1.5cm to about 2cm, or a total diametric wall thickness of about 3cm to about 4cm. The hollow channel inside the tubular balloon may have a diameter of about 1cm to about 3cm, optionally about 2cm. The diameter may depend on the degree of gastric restriction desired for slowing flow of chyme out of the stomach. For a pyloric plug device, the twisted portion of the frame may have an inner diameter of a few mm, for example, up to 5mm or less. For a tube device, the inner diameter of the twisted portion may be in the range of about 1 cm to about 3 cm, optionally about 2cm.

### Brief Description of the Drawings

Non-limiting embodiments of the invention are now described by way of example only, with reference to the accompanying drawings, in which:
Fig. 1 is a schematic side view of a bypass tube having a pyloric anchor;
Fig. 2 is a schematic side view showing detail of Fig. 1;
Figs. 3a and 3b are schematic side views illustrating how the anchor absorbs stomach and/or antral contractions;
Fig. 4 is a schematic side view of a pyloric anchor in a non-deployed configuration;
Fig. 5 is a schematic side view of the pyloric anchor of Fig. 4 in a .fully deployed configuration;
Fig. 6 is a schematic side view of the pyloric anchor of Figs. 4 and 5 in an implanted configuration; and
Fig. 7 is a schematic side view illustrating the anchor forming a pyloric plug.

### Detailed Description of Preferred Embodiments

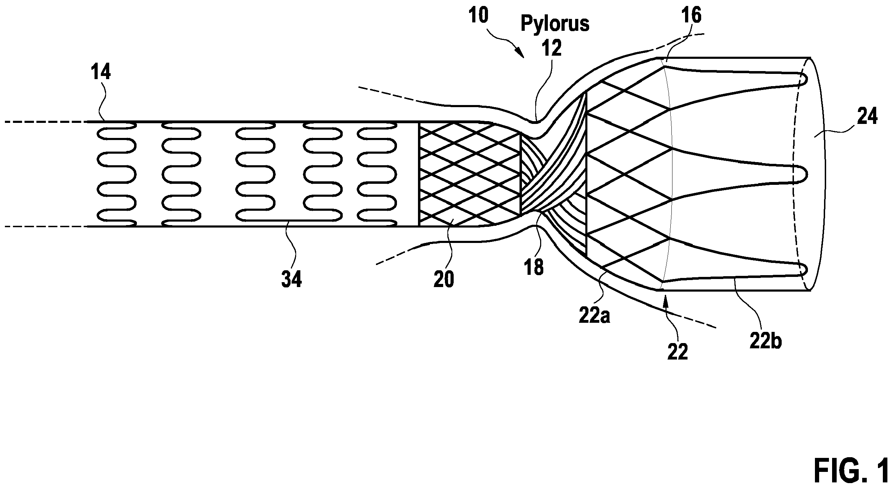

Referring to Figs. 1 to 6 of the drawings, a first embodiment of device for insertion into the gastro-intestinal tract comprises a pyloric anchor 10 for anchoring at or with respect to a pylorus 12 of a patient, and a bypass tube 14 extending distally from the anchor 10.

The pyloric anchor includes a self-deploying and/or self-expanding frame 16 including a first portion 18 at the pylorus, a second portion 20 in the form of a duodenal anchor, and a third portion 22 in the form of a gastric anchor. The gastric anchor serves to resist migration in a direction out of the stomach and through the pylorus 12. The duodenal anchor serves to resist migration from the duodenal side into the stomach.

The third portion 22 includes an annular shaped hub or collar 22a from which extend a plurality of independently deflectable limbs 22b. The gastric anchor further includes a tubular or torus balloon 24 having a shape suitable for seating in the pyloric antrum, such as by way of example, umbrella-shaped, or tulip-shaped. The balloon can serve as a gastric occupying component to reduce the volume of the stomach to food, and/or a gastric restriction with a passage dimensioned to restrict flow of chyme out of the stomach to the duodenum.

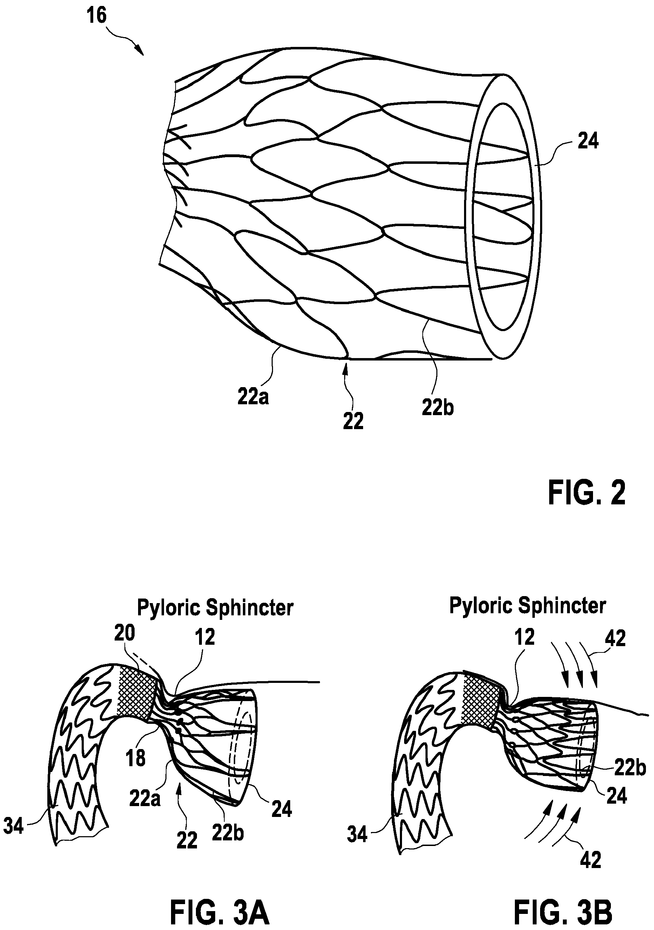

Referring to Fig. 3a, the third portion 22 of the frame serves to bias the balloon 24 into engagement with the stomach wall. Referring to Fig. 3b, during a stomach contraction, represented by arrows 42, the deflectable limbs 22b permit the mouth region of the gastric anchor to collapse locally at least partly inwardly, and thereby accommodate and/or absorb the stomach contraction without pulling or jerking the gastric anchor proximally. The hub or collar 22a is slightly stiffer, and provides a stable portion of the gastric anchor of large enough diameter to ensure that the gastric anchor will not pass accidentally through the pylorus even when the deflectable limbs 22b are being compressed inwardly.

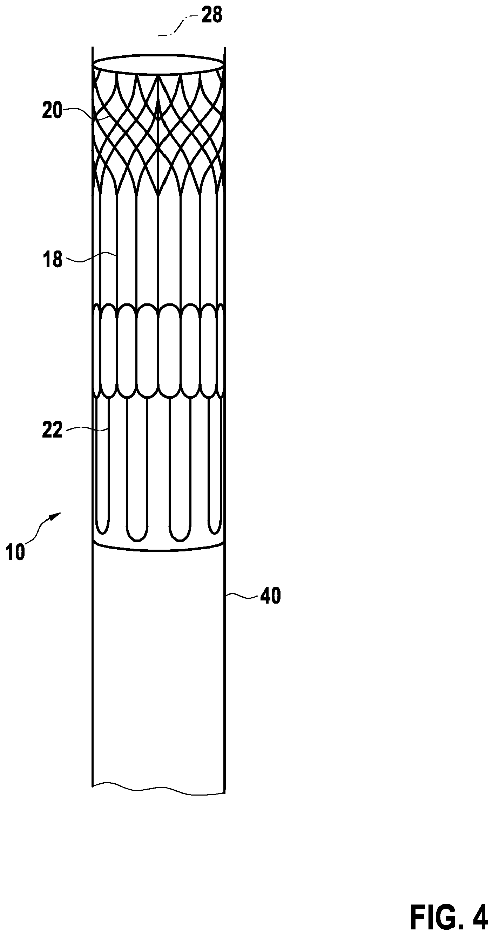

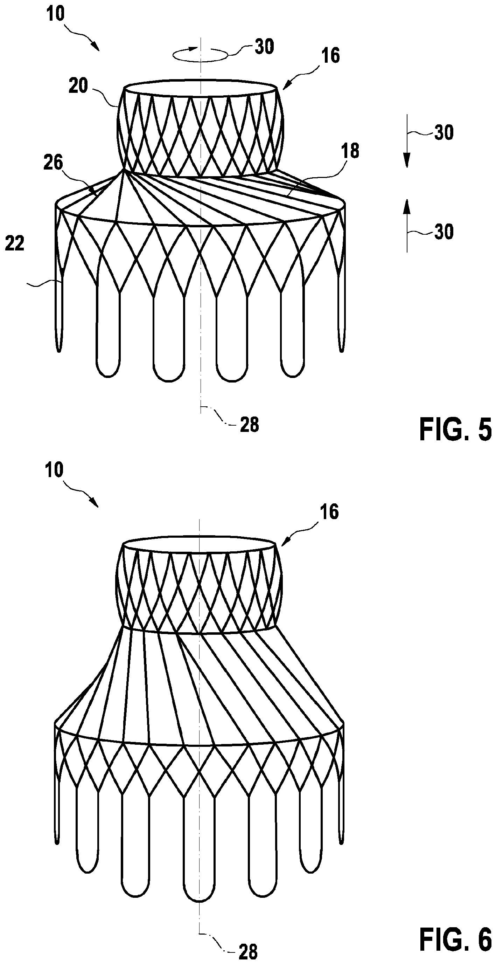

The second portion 20 forming a duodenal anchor may have a generally cylindrical shape, and/or have length not longer than the duodenal bulb. The second portion can be laser-cut or braided. Howsoever formed, in the illustrated embodiment, the first, second and third portions of the frame are optionally integral with one another and form a single body and/or unitary frame. Referring to Figs. 1 and 4 to 6, the frame 16 extends around an axis 28. The frame 16 is self-deployable from a non-deployed configuration (Fig. 4) towards a deployed (e.g. fully deployed) configuration (Fig. 5) for anchoring at the pylorus. In some embodiments, when implanted (Figs. 1 and 6), the frame 16 might not reach the fully deployed configuration, but instead achieve equilibrium with the pylorus and surrounding anatomy in a slightly compressed or implanted configuration in which the frame 16 continues to bias towards the fully deployed configuration.

In the illustrated form, the first portion 18 is configured to twist at least partly around the axis 28 when the frame transforms from the non-deployed configuration towards the deployed configuration. The first portion 18 comprise a plurality of struts extending predominantly in the same direction as each other to at least partly twist around the axis, and to torsionally couple the second portion 20 to the third portion 22. Twisting generates a rotational or torsional preload against the pylorus. This rotational preload resists axial migration by translating axial mechanical movement and/or force into rotational movement and/or force (as indicated by arrows 30). By this feature, relative displacement between the device and the anatomy can be reduced, so that the device substantially conforms to the anatomy even when the stomach antrum contracts. By virtue of the preload, the second and third portions can maintain a bias force against the pylorus from either side.

Referring to Fig. 1, the tube 14 comprises a support or reinforcement 34 present in and/or on the tube 14. The reinforcement is intended to prevent the flexible tube material from twisting or pinching or kinking within the small intestine. The reinforcement has a nonhelical shape, optionally comprising rows of repeating patterns (e.g. zig-zag or sinusoidal) extending circumferentially completely or partly around the tube circumference, to allow radial compression and expansion without substantial axial length changes, and interconnected by axial joining segments.

The frame 16, and the reinforcement 34, may be made of shape memory alloy, for example, nitinol. The frame 16 may be heatset in the deployed (e.g. fully deployed) and twisted configuration, and be deformable (e.g. compressible) to the non-deployed configuration for introduction using a delivery sheath 40. Upon removal of the sheath 40, the frame self-deploys towards the deployed configuration until implantation equilibrium is reached. The balloon 24 may be inflated to a suitable pressure to assist anchoring and occupy space within the stomach, but not too high that the inflation pressure will interfere with ability of the gastric anchor to accommodate or absorb stomach contractions.

A further feature is that the frame geometry is versatile in that can be configured for different applications. The degree of inward waisting of the first portion 18 can be controlled by the degree of twist around the frame axis 28. The greater the angle of twist, the narrower the waist. The degree of twisting can be one of the parameters used during heat setting to determine the shape of the frame 16 in the deployed configuration. For an anchor for a duodenal tube, as above for example, the frame can be configured with sufficient twist to form a waist between the first and second anchor regions, leaving open a'sufficiently large through-passage to allow passage of chyme.

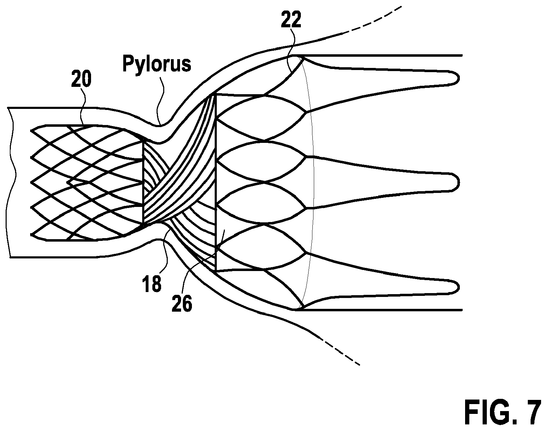

Referring to Fig. 7, for a pyloric plug, the frame can be configured with a large twisting angle around the axis to substantially pinch the centre of the frame closed. The plug embodiment may be used to complement a gastro-intestinal tract diversion, such as a gastro-jejunal diversion.

In both embodiments, the frame 16 may be coated, e.g. polymer coated, to form liner material 26, and/or to integrate with a wall of the balloon 24 (e.g. when used with a bypass tube). The liner material 26 can reduce any traumatic effect of the frame bearing against anatomical tissue. The liner material 26 could also be placed internally, specifically inside the first portion 18 of the frame, optionally creating bulky folds of the material by design when the frame is in the deployed configuration. This feature could be especially useful for a device in the form of a pyloric plug that occludes the pylorus as a complimentary device in the case of creation of gastro-jejunal diversion (not shown).

Although the above description and claims focus on certain features, protection is claimed for any novel feature or idea described herein and/or illustrated in the drawings, whether or not emphasis has been placed thereon.

## Claims

1. A gastric anchor (22) for anchoring a tube (14) with respect to a pylorus of a patient, the tube having a distal end for placement within the small intestine and a proximal end for placement near or at the pylorus, the gastric anchor (22) serving to prevent distal migration of the proximal end, the gastric anchor comprising a tubular balloon (24) and a self-expanding structure(22a, 22b), the gastric anchor having: a first end for seating on the gastric side of the pylorus; a first region (22a) having a first resistance to inward collapsing; and a second region (22b) having a second smaller resistance to inward collapsing, the first region being closer than the second region to the first end.

2. A gastric anchor according to claim 1, wherein the self-expanding structure (22a, 22b) provides at least partly the different first and second resistances to collapsing, the self-expanding structure comprising a first zone (22a) having a first zone resistance to inward collapsing, and a second zone (22b) with a smaller second zone resistance to inward collapsing.

3. A gastric anchor according to claim 2, wherein the second zone comprises a plurality of independently deflectable limbs or beams (22b) extending from the first zone.

4. A gastric anchor according to claim 2 or 3, wherein the first zone comprises an annular hub or collar (22a).

5. A gastric anchor according to any preceding claim, wherein the self-expandable structure (22a, 22b) is attached to or embedded within at least one of a radially inner wall and a radially outer wall of the tubular balloon (24), for biasing said wall of the tubular balloon outwardly.

6. A bypass tube (14) having a distal end for placement into the small intestine of a patient and a proximal end for placement near or at the pylorus, the bypass tube comprising a gastric anchor (22) for anchoring the proximal end of the tube with respect to the pylorus to prevent distal migration of the proximal end, the gastric anchor optionally according to any preceding claim, the gastric anchor comprising a tubular balloon (24) and a self-expanding structure (22a, 22b) for biasing a radially inner wall and/or a radially outer wall of the tubular balloon outwardly towards its enlarged form, the self-expanding structure comprising a first zone comprising an annular hub or collar (22a), and a second zone comprising a plurality of independently deflectable beams or limbs (22b) extending from the first zone.

7. A gastric anchor according to claim 4 or any claim dependent thereon, or a bypass tube according to claim 6, wherein the first zone (22a) comprises struts defining at least one circumferential row of cells.

8. A gastric anchor according to claim 3 or any claim dependent thereon, or a bypass tube according to claim 6 or any claim dependent thereon, wherein each independently deflectable limb or beam (22b) comprises:
- a single rib or strut; or
- plural struts collectively defining the beam or limb shape.

9. A gastric anchor according to claim 3 or any claim dependent thereon, or a bypass tube according to claim 6 or any claim dependent thereon, wherein each independently deflectable limb or beam (22b) extends to and/or towards a mouth region of the gastric anchor, to permit the mouth region to collapse local inwardly to accommodate stomach contractions.

10. A gastric anchor according to claim 1 or any claim dependent thereon, or a bypass tube according to claim 6 or any claim dependent thereon, wherein the tubular balloon (24) has a shape selected as one or more of: cup-shape; tulip-shape; bell-shape; pear-shape; tear-drop shape; funnel-shape; umbrella-shape.

11. A gastric anchor according to claim 1 or any claim dependent thereon, or a bypass tube according to claim 6 or any claim dependent thereon, wherein the tubular balloon (24) has one or more of:
a volume in the range of 200mL - 500 mL;
a radial thickness of about 1.5cm - about 2cm;
an inner diameter of between about 1cm to about 3cm, optionally about 2cm.

12. A gastric anchor according to claim 1 or any claim dependent thereon, or a bypass tube according to claim 6 or any claim dependent thereon, wherein:
a. the gastric anchor (22) has an outer diameter, in at least a portion of the anchor, in a range of about 4cm to about 6cm, optionally about 5cm; and/or
b. a duodenal side of a pyloric anchor has an outer diameter in a range of about 3cm to about 4cm.

13. A gastric anchor according to claim 1 or any claim dependent thereon, or a bypass tube according to claim 6 or any claim dependent thereon, wherein the self-expanding structure (22a, 22b) comprises a shape memory alloy, the alloy optionally containing nickel and titanium.

14. A bypass tube according to claim 6 or any claim dependent thereon, further comprising a duodenal anchor (20) for resisting migration through the pylorus in the direction of the stomach, wherein the gastric anchor and the duodenal anchor are coupled by a plurality of struts (18) that twist around the axis of the tube in the same direction of twist, thereby torsionally coupling together the gastric anchor (22) and the duodenal anchor (20).

15. A bypass tube according to claim 6 or any claim dependent thereon, wherein the tube has a radially collapsed compressed configuration for introduction, and a radially expanded configuration for functional implantation, the tube carrying a reinforcement element (34) for reinforcing the tube, the reinforcing element (34) having a non-helical shape, optionally wherein the non-helical shape comprises circumferentially expandable and collapsible rows interconnected by at least one substantially axial connecting segment.
